# EUROPÄISCHE PATENTSCHRIFT

(11) **EP 1 406 683 B1**
(45) Veröffentlichungstag und Bekanntmachung des Hinweises auf die Patenterteilung: **16.06.2010**
(21) Anmeldenummer: 02759855.6
(22) Anmeldetag: 17.07.2002
(51) Int. Cl.: A61M 1/00

(54) **VORRICHTUNG FUR DIE INTERMITTIERENDE OKKLUSION DES KORONARSINUS**
DEVICE FOR THE INTERMITTENT OCCLUSION OF THE CORONARY SINUS
DISPOSITIF D'OCCLUSION INTERMITTENTE DU SINUS CORONARIEN

(30) Priorität: 17.07.2001 AT 11132001
(43) Veröffentlichungstag der Anmeldung: 14.04.2004
(73) Patentinhaber: Miracor Medical Systems GmbH, 1090 Wien (AT)
(72) Erfinder: Mohl, Werner, 2571 Altenmarkt/Thennenberg (AT)
(74) Vertreter: Haffner, Thomas M.
(86) Internationale Anmeldenummer: PCT/AT2002/000212
(87) Internationale Veröffentlichungsnummer: WO 2003/008018

(56) Entgegenhaltungen:
- US-A- 4 493 697
- US-A- 4 689 041
- US-A- 4 934 996
- US-A- 5 024 668

## Beschreibung

Die Erfindung betrifft eine Vorrichtung für die intermittierende Okklusion des Koronarsinus mit einer Okklusions-Einrichtung zur Okklusion des Koronarsinus, einer Steuereinrichtung zum Auslösen und Aufheben der Okklusion und einer Druckmesseinrichtung zur Erfassung des Flüssigkeitsdrucks im Koronarsinus während der Okklusion, wobei eine in den Koronarsinus oder in eine in den Koronarsinus mündende Vene einbringbare und mit einem Perfusat beschickbare Leitung und eine mit einer Regelungseinrichtung verbundene Pumpe für das Perfusat vorgesehen ist, die Druckmesseinrichtung einen Speicher für Druckmesswerte aufweist und mit einer Recheneinheit zur Berechnung wenigstens einer aus den Druckmesswerten abgeleiteten Kenngröße verbunden ist, wobei die Regelung der eingebrachten Perfusatmenge unter Berücksichtigung der aus den Druckmesswerten abgeleiteten Kenngröße vorgenommen wird.

Eine derartige Vorrichtung ist aus der US 4,689,041 bekannt geworden.

Arterielles Blut, das den Herzmuskel versorgt, kann durch gesundes Herzgewebe hindurchtreten und es ernähren, hat jedoch Schwierigkeiten, das ischämische Gewebe zu erreichen. Dadurch wird die Versorgung des ischämischen Gewebes mit Nährstoffen sowie das Abführen von Abbauprodukten des Metabolismus von dem ischämischen Gewebe behindert. In diesem Zusammenhang ist bereits vorgeschlagen worden, das ischämische Gewebe durch retrograde Perfusion mit Blut zu versorgen. Die Retroinfusion von Blut in Koronarvenen spielt insbesondere im Bereich der Myokardprotektion während eines kurzfristigen Koronararterienverschlusses im Rahmen eines kardiologischen Eingriffes eine wichtige Rolle. Ein typischer derartiger Eingriff ist beispielsweise die Ballondilatation einer arteriosklerotisch verengten Koronararterie. Bei dieser auch als perkutane transluminale Koronarangioplastie (PTCA) bekannten Methode wird ein Ballonkatheter unter Röntgenkontrolle in den Bereich der Stenose der Koronararterie geführt und die arteriosklerotische Plaque durch Aufblasen des am Ende des Katheters befindlichen Ballons komprimiert. Während der Dilatation des Ballons findet stromabwärts in der Arterie keine Versorgung des Gewebes mit sauerstoffhaltigem Blut statt, wobei sich bereits bei Dilatationen von länger als 30 Sekunden Dauer funktionelle Veränderungen im Ischämiegebiet des Myokards feststellen lassen. Entsprechende Probleme der Ischämieprotektion des Myokards stellen sich auch bei anderen Eingriffen zur Koronarvaskularisierung wie z.B. bei Atherektomie, Koronarendoprothesen und Laseranwendungen.

Im Zusammenhang mit der kurzzeitigen Ischämieprotektion wird seit einiger Zeit eine Retroinfusion von arteriellem Blut oder anderen nutritiven Flüssigkeiten in eine Vene des betreffenden Ischämiegebietes des Myokards durchgeführt. Das Blut wird dabei über die entsprechende Vene in die nutritiven Kapillaren des Ischämiegebietes gepumpt und versorgt so das Myokard in dieser Region mit Sauerstoff und Substraten. Eine Vorrichtung zur Retroinfusion von Koronarvenen ist beispielsweise aus der US 4,934,996 bekanntgeworden, mit welcher eine druckgesteuerte intermittierende Koronarsinusokklusion vorgenommen werden kann. Die Vorrichtung umfasst eine Einrichtung zum Okkludieren des Sinus wie z.B. einen aufblasbaren Ballonkatheter, eine Druckmesseinrichtung zum Messen des Flüssigkeitsdruckes innerhalb des Koronarsinus und ein Steuergerät, das Auslösesignale für die Okklusions-Einrichtung erzeugt, um eine Okklusion auszulösen oder aufzuheben. Das Steuergerät ist hierbei derart ausgelegt, dass im Koronarsinus das Druckmaximum während jedes Herzschlages gemessen, ein Plateauwert der Druckmaxima aufeinanderfolgender Herzschläge rechnerisch abgeschätzt und die Okklusion des Koronarsinus auf Basis des Plateauwertes der Druckmaxima aufgehoben wird.

Das Okkludieren des Koronarsinus bewirkt einen Druckanstieg und in der Folge eine Retroperfusion von Blut über die entsprechende Vene in die nutritiven Kapillaren des Ischämiegebietes, sodass diese Gebiete mit Nährstoffen versorgt werden können. Bei Aufheben der Okklusion wird das retroperfundierte Blut ausgeschwemmt, wobei gleichzeitig die Abfallprodukte des Metabolismus abgeführt werden. Bei dem Verfahren gemäß der US 4,934,996 wird somit aufgrund der Messung des Druckmaximums im Koronarsinus während jedes Herzschlages rechnerisch eine systolische Druckkurve abgeschätzt, wobei die intermittierende Okklusion in Abhängigkeit von dem Plateauwert der systolischen Druckkurve gesteuert wird. Der Verlauf der abgeschätzten systolischen Druckkurve lässt auch einen Rückschluss auf die Leistungsfähigkeit des Herzens zu, wobei beispielsweise die Steigung der Kurve die Kontraktibilität des Herzens wiedergibt. Die Steigung der Kurve hat naturgemäß auch einen Einfluss auf die Höhe des Plateauwertes, wobei bei einer flacheren Kurve ein niedriger Plateauwert erreicht wird, wobei das Plateau überdies im Vergleich zu einem gesunden Herzen nach einer längeren Zeitspanne nach Einleiten der Okklusion erreicht wird. Eine Verschiebung der Kurve ergibt sich auch, wenn ein Koronargefäß bei einer interventionellen Handlung wie PTCA oder Stenting temporär oder durch eine Komplikation auch für länger verschlossen wird, sodass die Druckkurve langsamer ansteigt und auch länger braucht, bis das Plateau erreicht wird.

Die vorliegende Erfindung zielt nun darauf ab, eine Vorrichtung für die intermittierende Okklusion des Koronarsinus vorzuschlagen, welche auch bei einer durch den Verschluss eines Koronargefäßes hervorgerufenen Veränderung der Druckkurve eine ausreichende retrograde Versorgung des Ischämiegebietes mit Blut gewährleistet. Weiters soll im Vergleich zu den bekannten Vorrichtungen eine längere Intervention auch auf der arteriellen Seite gesetzt werden können, ohne dass es zu Gewebeinfarkten in den betroffenen Myokardregionen kommt. Die beispielsweise aus der US 4,887,608 bekannte diagnostische Komponente der intermittierenden Okklusion soll weiterhin aufrechtbleiben.

Die Erfindung steht hierbei in Zusammenhang mit einem Verfahren, bei dem in den okkludierten Koronarsinus oder wenigstens eine in den Koronarsinus mündende Vene ein Perfusat eingebracht wird, wobei die Regelung der eingebrachten Perfusatmenge unter Berücksichtigung wenigstens einer aus den Druckmesswerten abgeleiteten Kenngröße vorgenommen wird. Die erfindungsgemäße Vorrichtung weist eine Okklusions-Einrichtung zur Okklusion des Koronarsinus, eine Steuereinrichtung zum Auslösen oder Aufheben der Okklusion und eine Druckmesseinrichtung zur Erfassung des Flüssigkeitsdruckes im Koronarsinus während der Okklusion auf, wobei eine in den Koronarsinus oder in eine in den Koronarsinus mündende Vene einbringbare und mit einem Perfusat beschickbare Leitung und eine mit einer Regelungseinrichtung verbundene Pumpe für das Perfusat vorgesehen ist, die Druckmeseeinrichtung einen Speicher für Druckmesswerte aufweist und mit einer Recheneinheit zur Berechnung wenigstens einer aus den Druckmesswerten abgeleiteten Kenngröße verbunden ist, wobei die Regelung der eingebrachten Perfusatmenge unter Berücksichtigung der aus den Druckmesswerten abgeleiteten Kenngröße vorgenommen wird, und zeichnet sich dadurch aus, dass den im Speicher abgelegten Druckmesswerten ein Zeitstempel zugeordnet ist und der Speicher mit der Recheneinheit zur Berechnung der Ableitung der Flüssigkeitsdruckkurve nach der Zeit verbunden ist.

Durch Einbringen eines Perfusats wird das durch eine temporäre oder auch längere Verschließung eines Koronargefäßes durch interventionelle Handlungen wie PTCA oder Stenting oder durch eine Komplikation hervorgerufene Druckdefizit wieder wettgemacht. Dadurch kann in der Folge die durch den Koronargefäßverschluss flacher gewordene systolische bzw. diastolische Druckkurve wieder angehoben werden, sodass das Druckniveau einem Normal- oder Sollwert entspricht und eine ausreichende Retroperfusion in das Ischämiegebiet gewährleistet ist. Die eingebrachte Menge an Perfusat muss derart geregelt werden, dass weder ein zu niedriger noch ein zu hoher Retroinfusionsfluss erfolgt. Bei einem zu niedrigen Retroinfusionsfluas wäre der Venendruck zu niedrig, sodass eine ausreichende Versorgung des Myokards mit Sauerstoff nicht gewährleistet ist und die Myokardfunktion im Ischämiegebiet nicht aufrechterhalten werden kann. Bei zu hohem Infusionsfluss, d.h. bei einem zu hohen Koronarvenendruck, besteht jedoch die Gefahr, dass eine Überperfusion auftritt, welche die retrograde nutritive Kapillarfüllung nicht verbessert, sondern nur die Kontraktion des Myokards behindert und zu einem ineffektiven Abstrom des arteriellen Blutes in die systematische Zirkulation führt. Bei Retroinfusion mit einem zu hohen Koronarvenendruck besteht außerdem die Gefahr von irreversiblen Gefäßwandschädigungen.

Die Regelung der Perfusatmenge wird in Abhängigkeit von wenigstens einer aus den Druckmesswerten abgeleiteten Kenngröße vorgenommen, wobei hier mehrere Möglichkeiten denkbar sind. So kann beispielsweise ein fixer Grenzwert für die einzelnen Druckmesswerte vorgegeben werden, wobei bei Überschreiten dieser Grenzwerte die Menge an eingebrachtem Perfusat nicht mehr weiter erhöht wird. Innerhalb des okkludierten Koronarsinus steigen die lokalen Maxima der Druckmesswerte aufeinanderfolgender Herzschläge entsprechend einer Exponentialkurve an und nähern sich einem Plateauwert, sodass es sich schwierig gestaltet eine Regelgröße anzugeben, welche unabhängig ist von dem jeweiligen absoluten Druck, welcher im okkludierten Koronarsinus vorherrscht. Erfindungsgemäß ist daher vorgesehen dass den im Speicher abgelegten Druckmesswerten ein Zeitstempel zugeordnet ist und der Speicher mit der Recheneinheit zur Berechnung der Ableitung der Flüssigkeitsdruckkurve nach der zeit verbunden ist. Es wird also derart vorgegangen, dass als die aus den Druckmesswerten abgeleitete Kenngröße die Ableitung der Flüssigkeitsdruckkurve nach der Zeit gewählt wird. Dadurch wird nun eine Regelgröße vorgegeben, mit welcher es möglich wird, unabhängig vom jeweiligen absoluten Druckwert eine Regelung der eingebrachten Perfusatmenge vorzunehmen. Die zeitliche Ableitung der Druckkurve gibt hierbei die Steigung der Druckkurve wieder und erlaubt indirekt eine Aussage über das momentane Druckniveau und die Geschwindigkeit des Druckanstiegs innerhalb eines Herzschlages, sodass bei nicht aureichender Steilheit der Druckkurve der Perfusionsfluss erhöht werden muss, um an Werte zu kommen, die einem gesunden Herzen entsprechen und eine Kompensation des durch den verschluss eines Koronargefäßes verursachten Defizits arteriellen Blutflusses erlaubten. Die Berücksichtigung der zeitlichen Ableitung der Druckkurve als Regelgröße ist auch deshalb von Vorteil, als dadurch eine optimale Einstellung der Okklusionszeit möglich wird. Im Gegensatz zu anderen Verfahren zur Retroperfusion ist bei der erfindungsgemäßen Regelungsweise die okklusionszeit die Stellgröße und nicht der Gefäßinnendruck. Dabei soll sichergestellt sein, dass bei Erreichen der angestrebten Okklusionszeit der Gefäßinnendruck einen Plateauwert erreicht hat.

Bevorzugt kann im Rahmen der Erfindung auch so vorgegangen werden, dass als die aus den Druckmesswerten abgeleitete Kenngröße jeweils das innerhalb eines Herzschlages auftretende lokale Maximum und/oder lokale Minimum der Ableitung der Flüssigkeitsdruckkurve nach der Zeit gewählt wird. In diesem Fall ist die Vorrichtung derart weitergebildet, dass die Recheneinheit eine Vergleichsschaltung zur Ermittlung des jeweils innerhalb eines Herzschlages auftretenden lokalen Maximums und/oder lokalen Minimums der Ableitung der Flüssigkeitsdruckkurve nach der Zeit aufweist, wobei die Regelung der eingebrachten Perfusatmenge in Abhängigkeit von den lokalen Maxima und/oder lokalen Minima vorgenommen wird. Das lokale Maximum bzw. Minimum der zeitlichen Ableitung erlaubt eine präzise Beschreibung der Druckkurve und insbesondere der Steilheit des Druckanstiegs bzw. Druckabfalls, so dass auch dieser Kennwert sich sehr gut zur Regelung der eingebrachten Perfusatmenge eignet. Dadurch wird die Menge der in der Regeleinrichtung zu verarbeitenden bzw. auszuwertenden Daten verringert und trotzdem eine präzise Kurvenbeschreibung erreicht. Eine Weiterverarbeitung kann hierbei derart erfolgen, dass als die aus den Druckmesswerten abgeleitete Kenngröße der Plateauwert der lokalen Maxima und/oder der Plateauwert der lokalen Minima der Ableitung der Flüssigkeitsdruckkurve nach der Zeit gewählt wird. Die Vorrichtung ist in diesem Fall bevorzugt derart weitergebildet, dass die Recheneinheit eine Evaluierungsschaltung umfasst, welche einen Plateauwert der lokalen Druckmaxima und/oder lokalen Druckminima der Ableitung der Flüssigkeitsdruckkurve nach der Zeit rechnerisch abschätzt, und mit der Pumpe zur Regelung der eingebrachten Perfusatmenge in Abhängigkeit von dem Plateauwert zusammenwirkt. Diese bevorzugte Art der Regelung beruht somit auf dem Plateauwert der Extremwerte der zeitlichen Ableitung der Druckkurve, wobei dieser Plateauwert im wesentlichen zu einem Zeitpunkt erreicht wird, zu dem auch die systolische bzw. diastolische Druckkurve, einen Plateauwert erreicht. Der Plateauwert wird hierbei laufend anhand der aktuellen Druckwerte rechnerisch abgeschätzt.

Die Regelung erfolgt bevorzugt dadurch, dass die lokalen Maxima und/oder lokalen Minima der Ableitung der Flüssigkeitsdruckkurve nach der Zeit und/oder deren Plateauwerte mit einem Sollwert verglichen werden. Die Vorrichtung in dabei derart weitergebildet, dass die Recheneinheit eine Vergleichsschaltung zum Vergleich der lokalen Maxima und/oder lokalen Minima der Ableitung der Flüssigkeitsdruckkurve nach der Zeit und/oder deren Plateauwerte mit einem gespeicherten Sollwert umfasst. Der Sollwert kann beispielsweise ein einem gesunden Herzen entsprechender Wert sein oder auch ein Wert, welcher bei dem betreffenden Patienten vor dem Eingriff gemessen wurde.

Gemäß einer anderen Ausführung der Erfindung kann anstatt oder zusätzlich zur zeitlichen Ableitung der Druckkurve eine andere von den Druckmesswerten abgeleitete Kenngröße herangezogen werden, wozu derart vorgegangen wird, dass aus den Flüssigkeitsmesswerten ein Plateauwert der Druckminima und/oder ein Plateauwert der Druckmaxima aufeinanderfolgender Herzschläge rechnerisch abgeschätzt wird und als Kenngröße für die Regelung der eingebrachten Perfusatmenge herangezogen wird. Die Vorrichtung ist hierbei derart ausgebildet, dass die Druckmesseinrichtung einen Speicher für lokale Maxima der Druckmesswerte aufweist und die Recheneinheit eine Evaluierungsschaltung umfasst, welche einen Plateauwert der Druckmaxima aufeinanderfolgender Herzschläge rechnerisch abgeschätzt, und mit der Pumpe zur Regelung der eingebrachten Perfusatmenge in Abhängigkeit vom Plateauwert der Druckmaxima zusammenwirkt. Die Plateauwerte können hierbei in einfacher Weise aufgrund der Messwerte der Druckminima bzw. Druckmaxima abgeschätzt werden, wobei bevorzugt die rechnerische Abschätzung der Plateauwerte durch Einpassung der Druckmaxima bzw. -minima in eine Exponentialkurve vorgenommen wird. Die Abschätzung des jeweiligen Plateauwertes beruht somit auf einem mathematischen Näherungsverfahren, wobei der Plateauwert aufgrund der Druckmesswerte während jedes Herzschlages neu berechnet wird. Wird die systolische Druckkurve durch das zusätzliche Einbringen von Perfusat angehoben, steigt naturgemäß auch die diastolische Druckkurve, wobei in beiden Fällen nach einer bestimmten Zeit ein Plateauwert für die Druckmaxima und für die Druckminima erreicht wird. Wird nun der Infusionsfluss des Perfusats zu hoch gewählt, ergibt sich eine unverhältnismäßig starke Erhöhung des Plateauwertes der diastolischen Druckkurve relativ zur systolischen Druckkurve, sodass es aufgrund dieser Erkenntnis vorteilhaft erscheint, die Regelung der eingebrachten Perfusatmenge unter Berücksichtigung der Messwerte für die Druckminima und die Druckmaxima vorzunehmen.

Es kann somit nicht nur das systolische Plateau sondern auch das diastolische Plateau errechnet und der Trend der beiden Druckkurven zueinander ausgewertet werden, um das jeweilige Optimum der Okklusionszeit sowie des retrograden Infusionsflusses zu ermitteln. Wenn die optimale Menge des Perfusionsflusses überschritten wird, bleibt in der Regel der Plateauwert für die Druckmaxima konstant, wohingegen der Plateauwert der Druckminima weiter steigt. In diesem Sinne wird bevorzugt die Regelung der eingebrachten Perfusatmenge derart vorgenommen, dass die Differenz der Plateauwerte der Druckminima und der Druckmaxima berechnet wird und die Menge des eingebrachten Perfusats erhöht wird, solange die Differenz konstant bleibt oder größer wird. Die Vorrichtung ist dabei derart weitergebildet, dass die Regelungseinrichtung eine Vergleichsschaltung zum Vergleich der Plateauwerte der Druckminima und der Druckmaxima aufweist und mit der Pumpe zur Regelung der eingebrachten Perfusatmenge in Abhängigkeit von der Differenz der Druckmaxima und der Druckminima zusammenwirkt, wobei die Menge des eingebrachten Perfusats erhöht wird, solange die Differenz konstant bleibt oder größer wird. Der perfundierte Fluidfluss wird somit langsam erhöht und zwar solange die Differenz der Plateauwerte der Druckminima und der Druckmaxima konstant bleibt oder größer wird. Wenn dies nicht mehr der Fall ist, ist das Optimum des Perfusionsflusses erreicht, und es wird daher die Menge des eingebrachten Perfusats konstantgehalten.

Wie bereits erwähnt, wird durch die zusätzliche Einbringung eines Perfusats in den okkludierten Koronarsinus das durch einen Koronargefäßverschluss hervorgerufene Druckdefizit wettgemacht, sodass die systolische sowie die diastolische Druckkurve angehoben werden. Dadurch kommt es zu einer ausreichenden retrograden Versorgung des Ischämiegebietes mit Blut, und es wird weiters der Vorteil erreicht, dass eine längere Intervention auf der arteriellen Seite gesetzt werden kann, welche sonst wegen des Ausfalles eines zu großen Anteils der Perfusion nicht möglich wäre.

Neben der Regelung der eingebrachten Perfusatmenge ist auch die optimale Bestimmung der Okklusionszeiten von Bedeutung, und es wird in diesem Zusammenhang derart vorgegangen, dass die Druckmaxima aufeinanderfolgender Herzschläge mit dem Plateauwert der Druckmaxima verglichen werden und bei Erreichen eines vorbestimmten Prozentsatzes des Plateauwertes der Druckmaxima die Okklusion des Koronarsinus aufgehoben wird, wobei bevorzugt der Prozentsatz größer als 70 und kleiner als 98 gewählt wird. Die Vorrichtung ist hierbei bevorzugt derart weitergebildet, dass die Regelungseinrichtung mit der Steuereinrichtung zum Auslösen und Aufheben der Okklusion der Okklusions-Einrichtung verbunden ist, wobei die Okklusion aufgehoben wird, wenn die Druckmaxima aufeinanderfolgender Herzschläge einen vorbestimmten Prozentsatz des Plateauwertes der Druckmaxima erreichen, wobei der Prozentsatz größer als 70 und kleiner als 98 ist.

Bevorzugt können dem Perfusat Pharmazeutika zugesetzt werden. Derartige Pharmazeutika können beispielsweise Mittel zur Auflösung von Thromben aber auch therapeutische oder diagnostische Wirkstoffe z.B. Gerinnungshemmer, Kontrastmittel oder Beta-Blocker sein.

Bevorzugt wird das Perfusat unmittelbar in den Bereich der zu aktivierenden Gefäße eingebracht, zu welchem Zweck die mit einem Perfusat beschickbare Leitung relativ zur Okklusions-Einrichtung verschiebbar angeordnet ist und unmittelbar in den Bereich der zu aktivierenden Gefäße vorgeschoben werden kann. Dadurch kann das Perfusat gezielt am gewünschten Ort eingebracht werden, sodass eine Überwindung des Ventrikeldrucks direkt im Risikoareal, das heißt im zu aktivierenden Bereich, gelingt.

Vorteilhaft erscheint herbei, dass eine örtliche Trennung zwischen der Perfusateinbringung und der Okklusions-Einrichtung geschaffen wird. Der Perfusionskatheter kann beispielsweise bis in die Koronarvenenzirkulation vorgeschoben werden, wobei die Perfusion in Abhängigkeit von dem Perfusionswiderstand vorgenommen werden kann. Die Druckmessung erfolgt vorzugsweise im Bereich der Okklusions-Einrichtung, sodass eine örtliche Trennung zwischen Druckmessung und Einbringung des Perfusats erreicht wird und eine Beeinflussung der Druckmessung verhindert wird.

Eine Optimierung bzw. zusätzliche Kontrolle kann im Rahmen der Erfindung bevorzugt derart vorgenommen werden, dass zusätzlich der Flüssigkeitsdruck in einem arteriellen Gefäß des Herzens gemessen wird und die Regelung der eingebrachten Perfusatmenge zusätzlich unter Berücksichtigung der arteriellen Druckmesswerte vorgenommen wird, bzw. dass zusätzlich eine Druckmesseinrichtung zur Erfassung des Flüssigkeitsdrucks in einem arteriellen Gefäß des Herzens vorgesehen ist und die zusätzliche Druckmesseinrichtung mit der Recheneinheit verbunden ist, wobei die Regelung der eingebrachten Perfusatmenge zusätzlich unter Berücksichtigung der arteriellen Druckmesswerte vorgenommen wird. Dadurch kann die Regelung der eingebrachten Perfusatmenge nach Art einer closed loop vorgenommen werden, wobei durch den erweiterten Regelkreis zusätzliche Diagnose- und Steuerungsmöglichkeiten eröffnet werden.

Grundsätzlich können bezüglich der erfindungsgemäßen Vorrichtung die Steuereinrichtung, die Recheneinheit und die Regelungseinrichtung getrennte bauliche Einheiten darstellen, wobei jedoch alternativ die Steuereinrichtung die Regelungseinrichtung für die Pumpe und/oder die Recheneinheit umfassen kann.

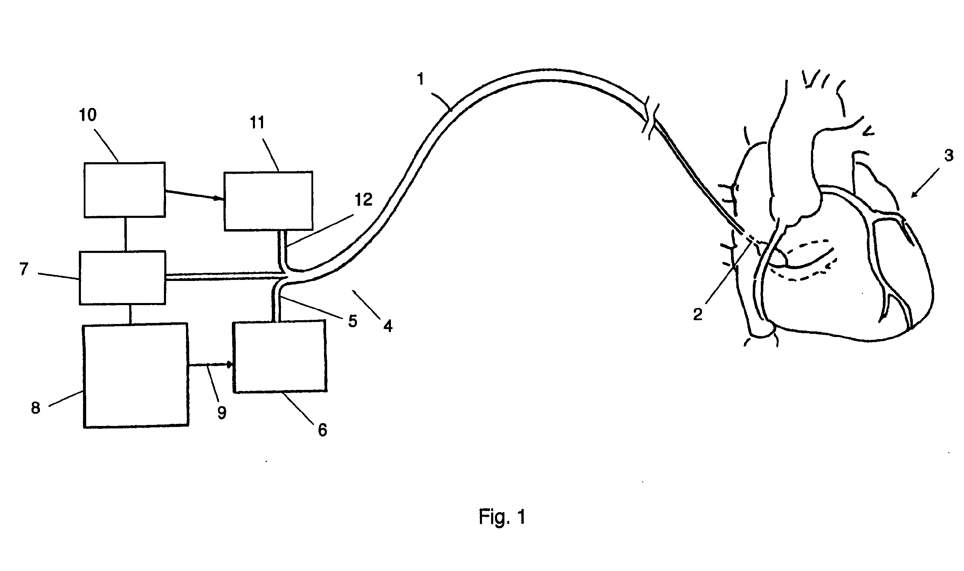

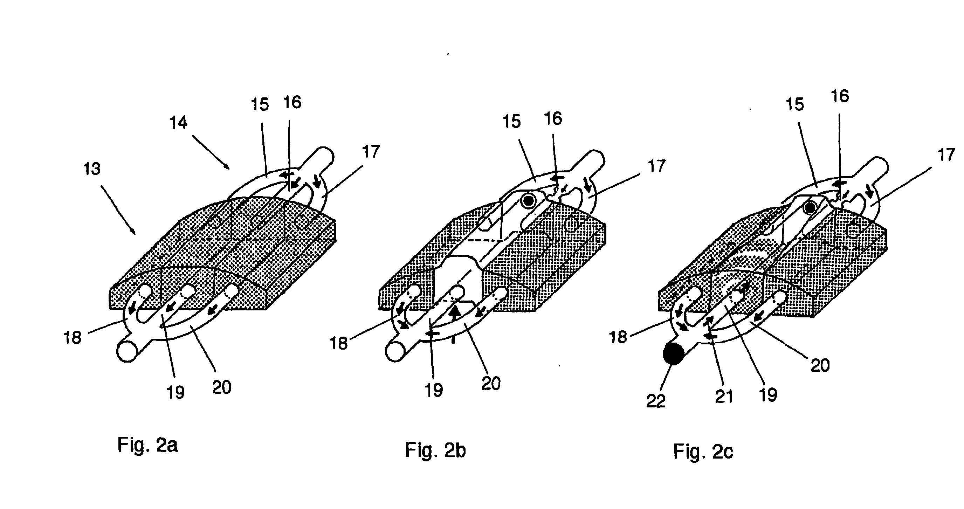

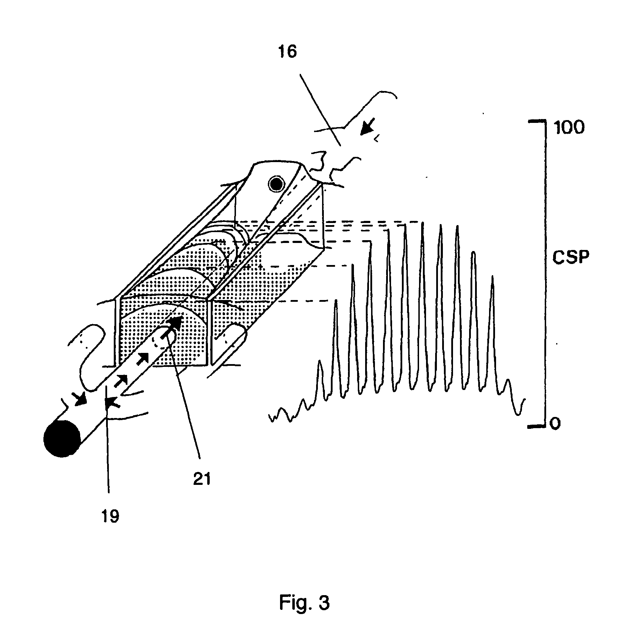

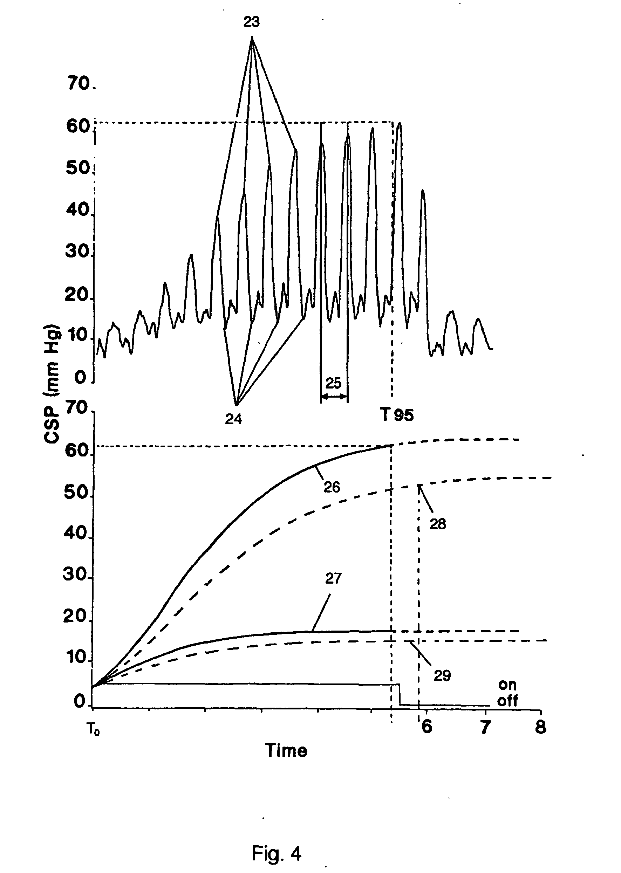

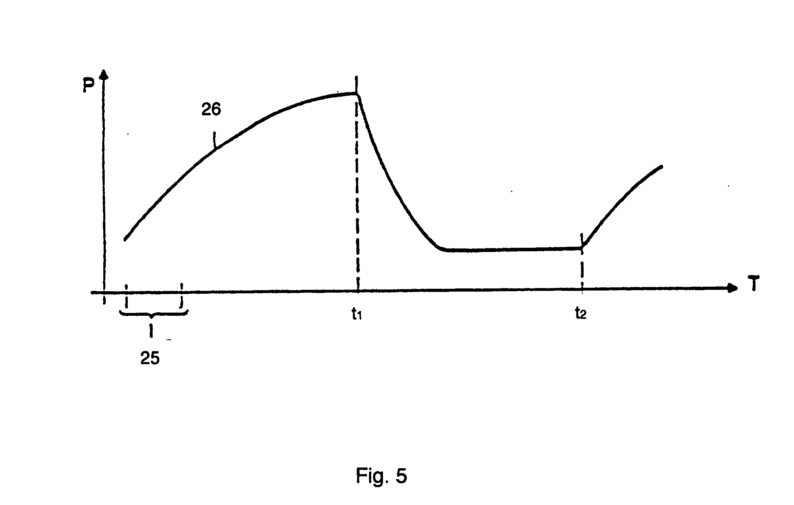

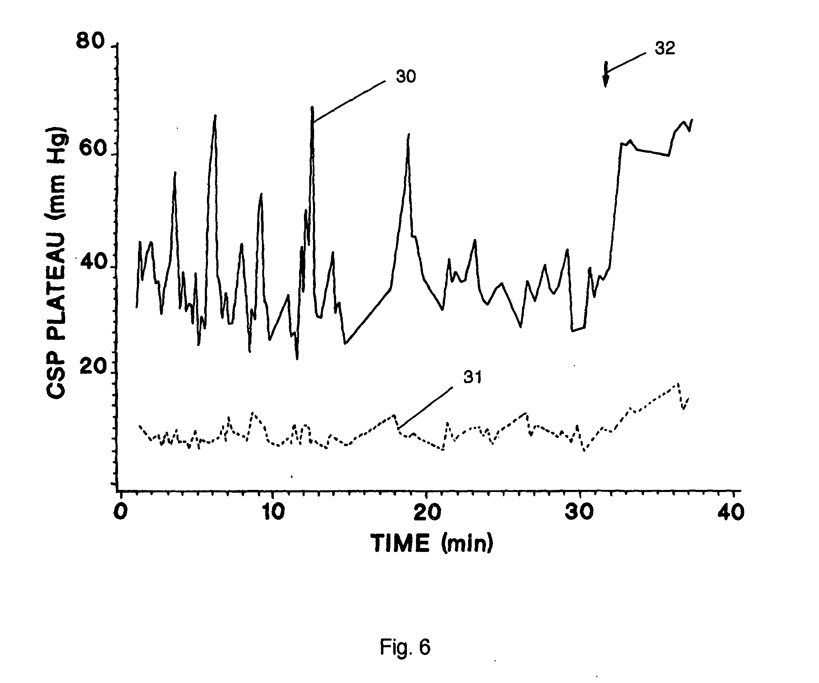

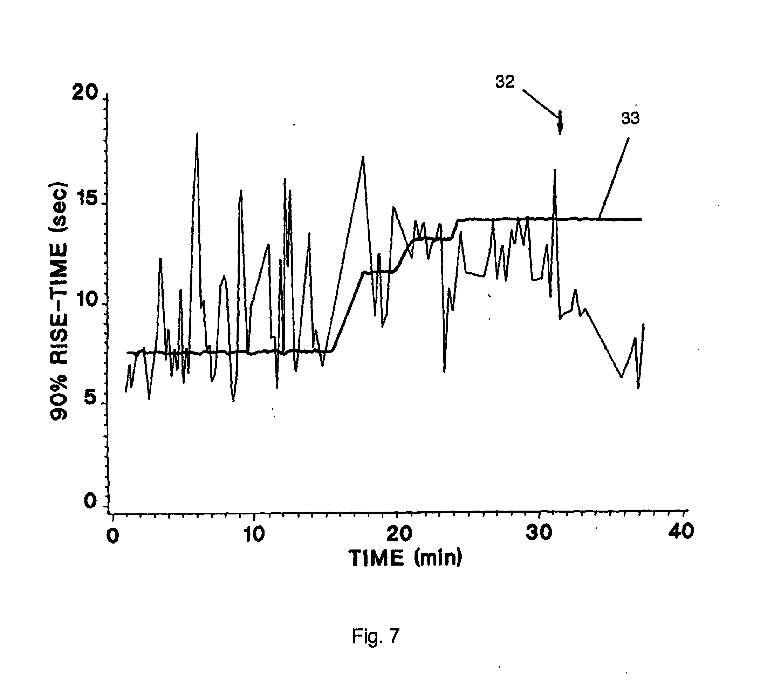

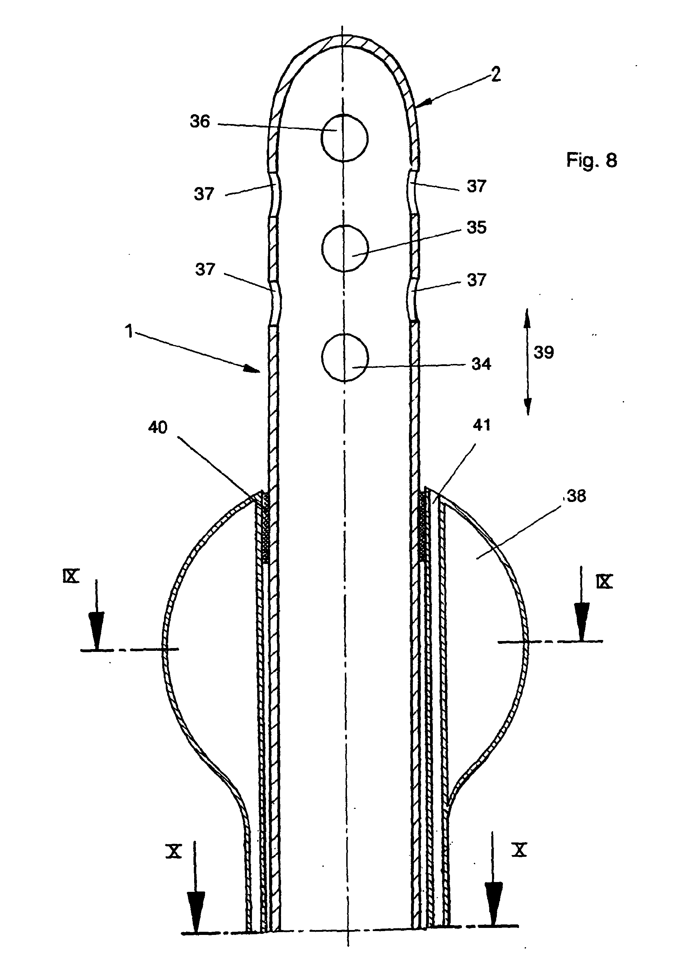

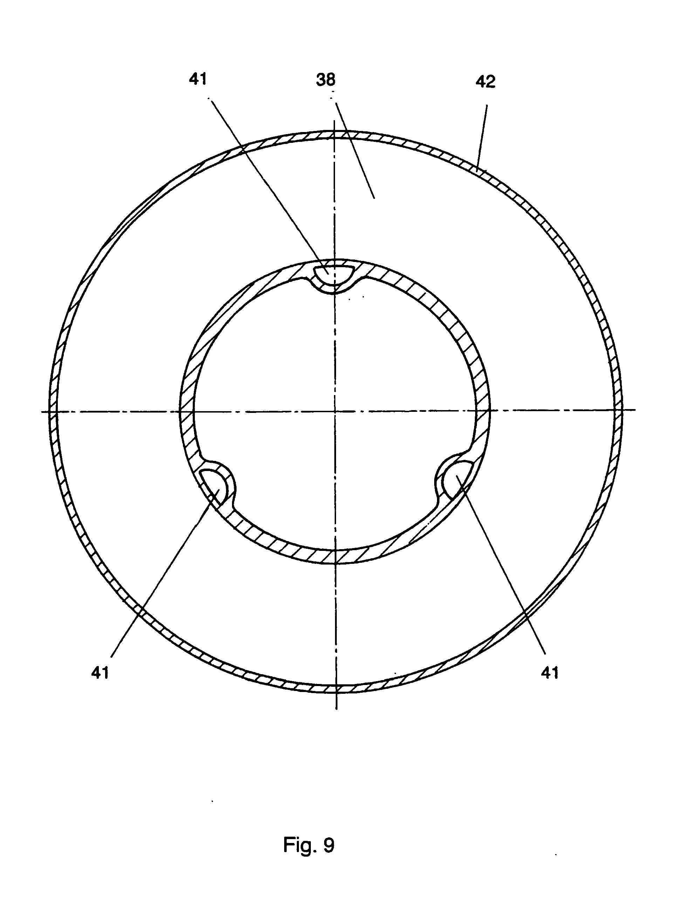

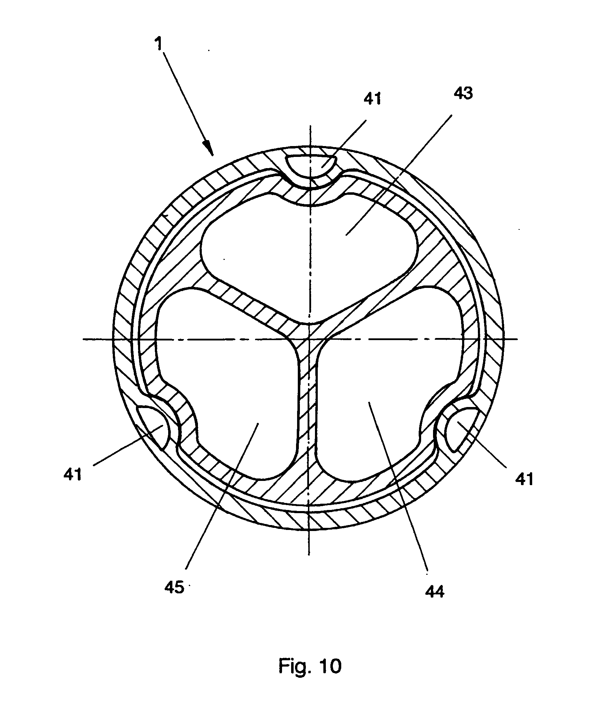

Die Erfindung wird nun nachfolgend an Hand einer Zeichnung näher erläutert. In dieser zeigen Figur 1 eine Diagrammansicht eines Herzens mit einer Vorrichtung zum intermittierenden Okkludieren des Koronarsinus, Figuren 2 und 3 vergleichende Darstellungen des Herzgewebes, Figuren 4 und 5 eine graphische Darstellung des Koronarsinusdruckverlaufes, Figur 6 den Verlauf des Druckplateaus, Figur 7 den Verlauf der Druckanstiegszeit, Figur 8 einen Schnitt durch das distale Ende eines zur Anwendung gelangenden Katheters mit einem aufgeweiteten Ballon, Figur 9 einen Schnitt nach der Linie IX-IX der Fig. 8 durch den aufgeweiteten Ballon und Figur 10 eine abgewandelte Ausbildung in einem Schnitt entsprechend der Linie X-X der Fig. 8.

In Fig. 1 ist schematisch die Vorrichtung zum intermittierenden Okkludieren des Koronarsinus dargestellt, wobei ein Multilumen-Katheter 1, dessen distales Ende 2 in den Koronarsinus des Herzens 3 über das Atrium eingesetzt wird, ersichtlich ist. Das proximale Ende 4 des Katheters 1 hat ein Ballonaufblaslumen 5, das mit einer Pumpe 6 verbunden ist. Der am distalen Ende 2 des Katheters 1 herrschende Druck wird von einer Druckmesseinrichtung 7 erfasst, wobei die Druckmesseinrichtung auch einen Speicher für die ermittelten Messwerte aufweist. Die jeweiligen Druckmesswerte werden einem Steuergerät 8 zugeführt, welches eine Schaltung enthält, um Steuersignale über die Leitung 9 zum Starten und Anhalten der Pumpe 6 zu liefern. Die Druckmesseinrichtung 7 ist weiters mit einer Regelungseinrichtung 10 verbunden, welche der Steuerung einer weiteren Pumpe 11 dient und die von der Pumpe 11 über ein weiteres Lumen 12 des Katheters 1 abgegebene Menge an Perfusat regelt.

Fig. 2 zeigt vergleichende Darstellung des Zustandes des Herzgewebes in unterschiedlichen Situationen. Fig. 2a zeigt hierbei die normale Situation, bei welcher das Herzgewebe 13 von der arteriellen Seite 14 über Arterien 15, 16 und 17 ausreichend versorgt wird. Die Venen sind schematische mit 18, 19 und 20 dargestellt. Fig. 2b zeigt den Zustand der Ischämie, bei welchem die mittlere Arterie 16 verschlossen oder verengt ist. Der entsprechende Gewebebereich wird unzureichend bzw. gar nicht mit Blut versorgt. Er erfolgt keine retrograde Perfusion in das ischämische Gebiet. In Fig. 2c ist nun die Situation dargestellt, welche sich bei Anwendung der Erfindung ergibt. Die Arterie 16 ist wiederum verschlossen, sodass eine ausreichende Versorgung des entsprechenden Gewebeteils nicht gegeben wäre.

Von der venöse Seite wird nun aber Blut in das ischämische Gebiet retroinfundiert, wie mit dem Pfeil 21 schematisch angedeutet ist, wobei mit 22 schematisch die Okklusion der Vene gezeigt ist.

Fig. 3 zeigt eine vergrößerte Darstellung gemäß Fig. 2c, in welcher der Druckverlauf in der Vene sowie der mit dem Druck zunehmende Rückstau des retroinfundierten Blutes in das ischämische Gebiet ersichtlich ist. Es ist wiederum die verschlossene Arterie 16 ersichtlich sowie die Vene 19, über welche das Perfusat in Richtung des Pfeils 21 in das Gewebe eindringt. Während der Okklusion kann in der okkludierten Vene der in Figur 3 rechts dargestellte Druckverlauf gemessen werden, wobei mit zunehmender Höhe der Druckmaxima mehr ischämisches Gebiet erreicht wird.

In Fig. 4 ist der von der Messeinrichtung 7 erfasste Druckverlauf dargestellt, wobei der Beginn der Okklusion mit T0 dargestellt ist. Es ist eine Reihe von systolischen Druckspitzen 23 und eine Reihe von diastolischen Tälern 24 ersichtlich. Die Pulsperiode 25 des Herzschlages wird durch die Zeit zwischen aufeinanderfolgenden Spitzen oder aufeinanderfolgenden Tälern dargestellt. Die systolischen Druckspitzen können in eine Exponentialkurve 26 der Formel Ps(t)=As(1-e^{-t/Ts}) eingepasst werden, worin As das asymptotische Plateau der Druckmaxima und Ts die Zeit bis zum Erreichen des Plateaus ist. In ähnlicher Weise können die diastolischen Drucktäler in eine Exponentialkurve 27 der Formel Pd(t)=Ad(1-e^{-t/Td}) eingepasst werden, worin Ad das asymptotische Plateau der Drucktäler und Td die Zeit zum Erreichen des Plateaus ist. Unter Annahme, dass die in Fig. 4 dargestellte systolische und diastolische Druckkurve 26 und 27 einem gesunden Herzen bzw. einem Sollwert entsprechen, können, wie mit 28 und 29 angedeutet, flachere Kurvenverläufe angegeben werden, welche der Situation entsprechen, in welcher ein Koronargefäß bei einer interventionellen Handlung temporär oder durch eine Komplikation auch für länger verschlossen wird. Es ist ersichtlich, dass die Plateauwerte der Kurven 28 und 29 niedriger sind, wobei das Plateau nach einer längeren Zeitspanne erreicht wird. Durch das erfindungsgemäße Einbringen von zusätzlichem Perfusat in den okkludierten Koronarsinus sollen nun die Kurven 28 und 29 auf das Niveau der Kurven 26 und 27 angehoben werden, sodass eine ausreichende retrograde Versorgung des Zielgebietes gewährleistet wird.

In Fig. 5 ist nun lediglich die systolische Druckkurve 16 dargestellt, wobei ersichtlich ist, dass bei Aufhebung der Okklusion bei t¹ die Kurve der lokalen Druckmaxima auf das Basisniveau zurückfällt bis zu dem Zeitpunkt t², bei welchem die Okklusion wiederum ausgelöst wird, worauf sich der Zyklus wiederholt.

In Fig. 6 ist über die gesamte Zeit eines Eingriffes an einer Koronararterie der Verlauf des Druckplateaus dargestellt. Mit 30 ist der Verlauf des systolischen und mit 31 der Verlauf des diastolischen Druckplateaus bezeichnet, wobei an der Stelle 32 der Eingriff beendet wird und der Verschluss der Koronararterie aufgehoben wird.

In Fig. 7 ist der Verlauf der Zeiten bis zum Erreichen von 90% des Druckplateaus ("90% rise time") über die gesamte Zeit eines Eingriffes dargestellt, wobei bei 33 der Mittelwert ersichtlich ist. Ausgehend von einem niedrigen Wert am Anfang des Eingriffes kann ein stufenweiser Anstieg bis zu einem Wert beobachtet werden, welcher nach Beendigung des Eingriffes bei 32 im wesentlichen konstant bleibt.

In den Figuren 8 bis 10 ist nun ein Multilumen-Ballonkatheter dargestellt, welcher im Rahmen des Verfahrens zum Einsatz gelangen kann.

In Fig. 8 ist das distale Ende eines Katheters 1 mit 2 bezeichnet. Je nach Anzahl der Lumina können unterschiedliche Austrittsöffnungen 34, 35, 36 oder 37 mit voneinander verschiedenen Lumina verbunden sein, sodass auf diese Weise die gesonderte Zuführung von pharmakologischen Substanzen oder die Zuführung eines Perfusats ebenso gelingt, wie beispielsweise die Ermittlung von Druckmesswerten über die in einem Lumen enthaltene Flüssigkeitssäule. Das distale Ende des Katheters 2 ist in einem aufweitbaren Ballon 38 in Richtung des Doppelpfeils 39 gleitend und dichtend geführt, wobei die Dichtung mit 40 bezeichnet ist. Weiters ist ein weiteres Lumen 41 für eine Druckmessung ersichtlich.

Nach Erreichen einer vorbestimmten definierten Position des Ballons kann der Ballon 38 aufgeweitet werden, worauf die jeweils geeignete Position des distalen Endes 2 bzw. der Austrittsöffnungen bzw. Mündungen 34, 35, 36 oder 37 durch axiale Verschiebung des distalen Endes 2 relativ zum Ballon 38 in Richtung des Doppelpfeils 39 eingestellt werden kann. Wenn das distale Ende bei entsprechender Wahl der Abstände der Durchbrechungen 34, 35, 36 oder 37 in einer Weise zurückgezogen wird, dass ein Teil der Mündungen bzw. Durchbrechungen dichtend vom Ballon abgedeckt ist, bleiben lediglich die freien Durchtrittsöffnungen bzw. Durchbrechungen 35, 36 oder 37 in unmittelbarem Kontakt mit dem umgebenden Fluid und insbesondere mit dem Blut im Blutkreislauf eines Blutgefäßes.

In der Darstellung nach Fig. 9 ist lediglich der Ballon 38 dargestellt, dessen Außenhaut mit 42 bezeichnet ist. Die dem verschieblichen Teil des Katheters benachbarte Innenwand trägt axiale Lumina 41, über welche beispielsweise Druckmesswerte gewonnen werden können. Über einen oder mehrere derartiger axialer Kanäle 41 kann auch das Druckmittel für das Aufweiten des Ballons 38 zugeführt werden.

Bei der Darstellung nach Fig. 10 ist ein mehrlumiger Katheter 1 dargestellt, dessen einzelne Lumina mit 43, 44 und 45 bezeichnet sind. Die einzelnen Lumina können mit unterschiedlichen Mündungen bzw. Durchbrechungen 34, 35, 36 bzw. 37, wie sie in Fig. 8 ersichtlich sind, verbunden sein, wobei in derartigen Lumina auch Drähte zur elektrischen Kontaktierung von Messsensoren angeordnet sein können.

## Patentansprüche

1. Vorrichtung für die intermittierende Okklusion des Koronarsinus mit einer Okklusions-Einrichtung (38) zur Okklusion des Koronarsinus, einer Steuereinrichtung (8) zum Auslösen und Aufheben der Okklusion und einer Druckmesseinrichtung (7) zur Erfassung des Flüssigkeitsdrucks im Koronarsinus während der Okklusion, wobei eine in den Koronarsinus oder in eine in den Koronarsinus mündende Vene einbringbare und mit einem Perfusat beschickbare Leitung und eine mit einer Regelungseinrichtung (10) verbundene Pumpe (11) für das Perfusat vorgesehen ist, die Druckmesseinrichtung (7) einen Speicher für Druckmesswerte aufweist und mit einer Recheneinheit zur Berechnung wenigstens einer aus den Druckmesswerten abgeleiteten Kenngröße verbunden ist, wobei die Regelung der eingebrachten Perfusatmenge unter Berücksichtigung der aus den Druckmesswerten abgeleiteten Kenngröße vorgenommen wird, **dadurch gekennzeichnet, dass** den im Speicher abgelegten Druckmesswerten ein Zeitstempel zugeordnet ist und der Speicher mit der Recheneinheit zur Berechnung der Ableitung der Flüssigkeitsdruckkurve nach der Zeit verbunden ist.

2. Vorrichtung nach Anspruch 1, **dadurch gekennzeichnet, dass** die Recheneinheit eine Vergleichsschaltung zur Ermittlung des jeweils innerhalb eines Herzschlages auftretenden lokalen Maximums und/oder lokalen Minimums der Ableitung der Flüssigkeitsdruckkurve nach der Zeit aufweist, wobei die Regelung der eingebrachten Perfusatmenge in Abhängigkeit von den lokalen Maxima und/oder lokalen Minima vorgenommen wird.

3. Vorrichtung nach Anspruch 2, **dadurch gekennzeichnet, dass** die Recheneinheit eine Evaluierungsschaltung umfasst, welche einen Plateauwert der lokalen Druckmaxima und/oder lokalen Druckminima der Ableitung der Flüssigkeitsdruckkurve nach der Zeit rechnerisch abschätzt, und mit der Pumpe zur Regelung der eingebrachten Perfusatmenge in Abhängigkeit von dem Plateauwert zusammenwirkt.

4. Vorrichtung nach Anspruch 2 oder 3, **dadurch gekennzeichnet, dass** die Recheneinheit eine Vergleichsschaltung zum Vergleich der lokalen Maxima und/oder lokalen Minima der Ableitung der Flüssigkeitsdruckkurve nach der Zeit und/oder deren Plateauwerte mit einem gespeicherten Sollwert umfasst.

5. Vorrichtung nach einem der Ansprüche 1 bis 4, **dadurch gekennzeichnet, dass** die Druckmesseinrichtung einen Speicher für lokale Maxima der Druckmesswerte aufweist und die Recheneinheit eine Evaluierungsschaltung umfasst, welche einen Plateauwert der Druckmaxima aufeinanderfolgender Herzschläge rechnerisch abgeschätzt, und mit der Pumpe zur Regelung der eingebrachten Perfusatmenge in Abhängigkeit vom Plateauwert der Druckmaxima zusammenwirkt.

6. Vorrichtung nach Anspruch 5, **dadurch gekennzeichnet, dass** die Recheneinheit eine Vergleichsschaltung zum Vergleich der Plateauwerte der Druckminima und der Druckmaxima aufweist und mit der Pumpe zur Regelung der eingebrachten Perfusatmenge in Abhängigkeit von der Differenz der Druckmaxima und der Druckminima zusammenwirkt, wobei die Menge des eingebrachten Perfusats erhöht wird, solange die Differenz konstant bleibt oder größer wird.

7. Vorrichtung nach einem der Ansprüche 1 bis 6, **dadurch gekennzeichnet, dass** die Regelungseinrichtung (10) mit der Steuereinrichtung (8) zum Auslösen und Aufheben der Okklusion der Okklusions-Einrichtung verbunden ist, wobei die Okklusion aufgehoben wird, wenn die Druckmaxima aufeinanderfolgender Herzschläge einen vorbestimmten Prozentsatz, vorzugsweise größer als 70 und kleiner als 98 Prozent, des Plateauwertes der Druckmaxima erreichen.

8. Vorrichtung nach einem der Ansprüche 1 bis 7, **dadurch gekennzeichnet, dass** die Evaluierungsschaltung die rechnerische Abschätzung der Plateauwerte durch Einpassung der Druckmaxima bzw. -minima in eine Exponentialkurve vornimmt.

9. Vorrichtung nach einem der Ansprüche 1 bis 8, **dadurch gekennzeichnet, dass** die Steuereinrichtung (8) die Regelungseinrichtung (10) für die Pumpe und/oder die Recheneinheit umfasst.

10. Vorrichtung nach einem der Ansprüche 1 bis 9, **dadurch gekennzeichnet, dass** die mit einem Perfusat beschickbare Leitung relativ zur Okklusions-Einrichtung (38) verschiebbar angeordnet ist und unmittelbar in den Bereich der zu aktivierenden Gefäße vorgeschoben werden kann.

11. Vorrichtung nach einem der Ansprüche 1 bis 10, **dadurch gekennzeichnet, dass** zusätzlich eine Druckmesseinrichtung zur Erfassung des Flüssigkeitsdrucks in einem arteriellen Gefäß des Herzens vorgesehen ist und die zusätzliche Druckmesseinrichtung mit der Recheneinheit verbunden ist, wobei die Regelung der eingebrachten Perfusatmenge zusätzlich unter Berücksichtigung der arteriellen Druckmesswerte vorgenommen wird.

## Claims

1. A device for the intermittent occlusion of the coronary sinus including an occlusion means (38) for the occlusion of the coronary sinus, a control means (8) for the triggering and releasing of said occlusion and a pressure measuring means (7) for determining the fluid pressure prevailing in the coronary sinus during the occlusion, wherein a duct capable of being introduced into the coronary sinus, or a vein running into the coronary sinus, and capable of being charged with a perfusate as well as a perfusate pump (11) connected with a control device (10) are provided and wherein the pressure measuring means (7) comprises a memory for the measured pressure values and is connected with a computation unit for the calculation of at least one parameter derived from said measured pressure values, wherein the control of the introduced amount of perfusate is performed under consideration of said parameter derived from the measured pressure values **characterized in that** the measured pressure values stored in the memory are assigned time stamps and the memory is connected with the computation unit for the calculation of the derivation of the fluid pressure curve according to time.

2. A device according to claim 1, **characterized in that** the computation unit comprises a comparator circuit for the determination of the local maximum and/or local minimum each occurring within a heartbeat, of the derivation of the fluid pressure curve according to time, wherein the control of the introduced amount of perfusate is performed as a function of the local maxima and/or local minima.

3. A device according to claim 2, **characterized in that** the computation unit comprises an evaluation circuit which computationally estimates a plateau value of the local pressure maxima and/or local pressure minima of the derivation of the fluid pressure curve according to time and cooperates with the pump for the control of the introduced amount of perfusate as a function of the plateau value.

4. A device according to claim 2 or 3, **characterized in that** the computation unit comprises a comparator circuit for the comparison of the local maxima and/or local minima of the derivation of the fluid pressure curve according to time, and/or the plateau values thereof, with a stored set value.

5. A device according to any one of claims 1 to 4, **characterized in that** the pressure measuring means comprises a memory for local maxima of the measured pressure values and the computation unit comprises an evaluation circuit computationally estimating a plateau value of the pressure maxima of consecutive heartbeats and cooperates with the pump for the control of the introduced amount of perfusate as a function of the plateau value of the pressure maxima.

6. A device according to claim 5, **characterized in that** the computation unit comprises a comparator circuit for the comparison of the plateau values of the pressure minima and pressure maxima and cooperates with the pump for the control of the introduced amount of perfusate as a function of the difference between the pressure maxima and pressure minima, whereby the amount of perfusate introduced is raised as long as the difference remains constant or increases.

7. A device according to any one of claims 1 to 6, **characterized in that** the control device (10) is connected with the control means (8) for the triggering and releasing the occlusion by the occlusion device, wherein the occlusion is released as the pressure maxima of consecutive heartbeats have reached a predetermined percentage, preferably larger than 70 and smaller than 98%, of the plateau value of the pressure maxima.

8. A device according to any one of claims 1 to 7, **characterized in that** the evaluation circuit performs the computational estimation of the plateau values by the insertion of the pressure maxima or minima into an exponential curve.

9. A device according to any one of claims 1 to 8, **characterized in that** the control means (8) comprises the control device (10) for the pump and/or the computation unit.

10. A device according to any one of claims 1 to 9, **characterized in that** the duct capable of being charged with a perfusate is arranged to be displaceable relative to the occlusion device (38) and capable of being pushed forward directly into the region of the vessels to be activated.

11. A device according to any one of claims 1 to 10, **characterized in that** an additional pressure measuring means is provided for the determination of the fluid pressure in an arterial heart vessel and the additional pressure measuring means is connected with the computation unit, whereby the control of the introduced amount of perfusate is effected under additional consideration of the arterial pressure values measured.

## Revendications

1. Dispositif pour l'occlusion intermittente du sinus coronaire, comportant un dispositif d'occlusion (38) pour l'occlusion du sinus coronaire, un dispositif de commande (8) destiné à déclencher et à arrêter l'occlusion et un manomètre (7) pour enregistrer la pression d'un fluide dans le sinus coronaire pendant l'occlusion, dans lequel il est prévu une conduite, propre à être implantée dans le sinus coronaire ou dans une veine débouchant dans le sinus coronaire et pouvant être remplie par un perfusat, et une pompe (11) pour le perfusat, reliée à un dispositif de réglage (10), le manomètre (7) comporte une mémoire pour les valeurs de pression mesurées et est relié à une unité de calcul destinée à calculer au moins l'une des grandeurs caractéristiques dérivées des valeurs de pression mesurées, le réglage de la quantité de perfusat introduite étant effectué en tenant compte des grandeurs caractéristiques dérivées des valeurs de pression mesurées, **caractérisé en ce qu'**un horodatage est associé aux valeurs de pression mesurées, stockées dans la mémoire, et la mémoire est reliée à l'unité de calcul pour calculer la dérivée de la courbe de pression du liquide en fonction du temps.

2. Dispositif selon la revendication 1, **caractérisé en ce que** l'unité de calcul comporte un composant de comparaison destiné à déterminer le maximum local et/ou le minimum local, apparaissant dans chaque cas lors d'un battement cardiaque, de la dérivée de la courbe de pression du liquide en fonction du temps, le réglage de la quantité de perfusat introduite étant effectué en fonction des maxima locaux et/ ou des minima locaux.

3. Dispositif selon la revendication 2, **caractérisé en ce que** l'unité de calcul comporte un composant d'évaluation, qui effectue une évaluation numérique d'une valeur plateau des maxima de pression locaux et/ou des minima de pression locaux de la dérivée de la courbe de pression du liquide en fonction du temps, et coopère avec la pompe pour le réglage de la quantité de perfusat introduite en fonction de la valeur plateau.

4. Dispositif selon la revendication 2 ou 3, **caractérisé en ce que** l'unité de calcul comporte un composant de comparaison destiné à comparer à une valeur de consigne, stockée en mémoire, les maxima locaux et/ou les minima locaux de la dérivée de la courbe de pression du liquide en fonction du temps et/ ou les valeurs plateaux de ceux-ci.

5. Dispositif selon l'une quelconque des revendications 1 à 4, **caractérisé en ce que** le manomètre comporte une mémoire pour les maxima locaux des valeurs de pression mesurées et l'unité de calcul comporte un composant d'évaluation, qui effectue une évaluation numérique d'une valeur plateau des maxima de pression des battements cardiaques consécutifs, et coopère avec la pompe pour le réglage de la quantité de perfusat introduite en fonction de la valeur plateau des maxima de pression.

6. Dispositif selon la revendication 5, **caractérisé en ce que** l'unité de calcul comporte un composant de comparaison destiné à comparer les minima de pression et les maxima de pression et coopère avec la pompe pour le réglage de la quantité de perfusat introduite en fonction de la différence entre les maxima de pression et les minima de pression, la quantité de perfusat étant augmentée tant que la différence reste constante ou devient plus grande.

7. Dispositif selon l'une quelconque des revendications 1 à 6, **caractérisé en ce que** le dispositif de réglage (10) est relié au dispositif de commande (8) destiné à déclencher et arrêter l'occlusion du dispositif d'occlusion, l'occlusion étant arrêtée lorsque les maxima de pression des battements cardiaques consécutifs atteignent un pourcentage prédéterminé, de préférence supérieur à 70 et inférieur à 98 pour cent, de la valeur plateau des maxima de pression.

8. Dispositif selon l'une quelconque des revendications 1 à 7, **caractérisé en ce que** le composant d'évaluation procède à l'évaluation numérique des valeurs plateaux par l'ajustement des maxima de pression ou minima de pression dans une courbe exponentielle.

9. Dispositif selon l'une quelconque des revendications 1 à 8, **caractérisé en ce que** le dispositif de commande (8) comporte le dispositif de réglage (10) pour la pompe et/ou l'unité de calcul.

10. Dispositif selon l'une quelconque des revendications 1 à 9, **caractérisé en ce que** la conduite, propre à contenir un perfusat, est disposée de manière mobile par rapport au dispositif d'occlusion (38) et peut être avancée directement dans la zone des vaisseaux à activer.

11. Dispositif selon l'une quelconque des revendications 1 à 10, **caractérisé en ce qu'**il est prévu, en plus, un manomètre destiné à enregistrer la pression du liquide dans un vaisseau artériel du coeur, et le manomètre supplémentaire est relié à l'unité de calcul, le réglage de la quantité de perfusat introduite étant effectué, en plus, en tenant compte des valeurs de pression artérielle mesurées.
